# EUROPEAN PATENT APPLICATION

(11) **EP 3 660 147 A1**
(43) Date of publication of application: **03.06.2020**
(21) Application number: 18382872.2
(22) Date of filing: 30.11.2018
(51) Int. Cl.: C12N 9/08, C12P 7/00

(54) **PROCESS FOR SELECTIVE SYNTHESIS OF 4-HYDROXYISOPHORONE AND 4-KETOISOPHORONE BY FUNGAL PEROXYGENASES**

(71) Applicant: Consejo Superior de Investigaciones Científicas (CSIC), 28006 Madrid (ES)
(72) Inventor: ARANDA OLIDEN, Mª del Carmen, 41012 Sevilla (ES); DEL RÍO ANDRADE, José Carlos, 41012 Sevilla (ES); MARTÍNEZ FERRER, Ángel Tomás, 28040 Madric (ES); GUTIÉRREZ SUAREZ, Ana, 41012 Sevilla (ES)
(74) Representative: Pons Ariño, Angel

(57) **Abstract**

The process of the present invention relates to the biocatalytic transformation of isophorone (3,5,5-trimethylcyclohex-2-en-1-one) and 4-hydroxyisophorone by several unspecific peroxygenases produced by fungi. These biocatalysts, that only require H₂O₂ or organic peroxides for activation, potentially represent a novel simple and environmentally friendly route for the production of isophorone derivatives, such as the high value products for the pharmaceutical and flavour and fragrance industry 4-hydroxyisophorone and 4-ketoisophorone.

SEQUENCE LISTING

<110> Consejo Superior de Investigaciones Científicas (CSIC)

<120> PROCESS FOR SELECTIVE SYNTHESIS OF 4-HYDROXYISOPHORONE AND
      4-KETOISOPHORONE BY FUNGAL PEROXYGENASES

<130> EP1641.1440

<160> 3

<170> PatentIn version 3.5

<210> 1
<211> 259
<212> PRT
<213> Chaetomium globosum

<400> 1

Met Arg Thr Ser Leu Leu Pro Ala Leu Ala Ala Val Ser Pro Val Leu
1               5                   10                  15

Ala Gly Phe Asp Thr Trp Ala Pro Pro Gly Pro Tyr Asp Val Arg Gly
            20                  25                  30

Pro Cys Pro Met Leu Asn Thr Leu Thr Asn His Gly Phe Phe Pro His
        35                  40                  45

Asp Gly Gln Asp Ile Asp Arg Glu Thr Thr Glu Asn Ala Leu Phe Asp
    50                  55                  60

Ala Leu His Val Asn Lys Thr Leu Ala Ser Phe Leu Phe Asp Phe Ala
65                  70                  75                  80

Leu Thr Thr Asn Pro Ile Ala Asn Ser Thr Thr Phe Ser Leu Asn Asp
                85                  90                  95

Leu Gly Asn His Asn Val Leu Glu His Asp Ala Ser Leu Ser Arg Ala
            100                 105                 110

Asp Ala Tyr His Gly Ser Val Leu Ala Phe Asn His Thr Ile Phe Glu
        115                 120                 125

Glu Thr Lys Ser Tyr Trp Thr Asp Glu Thr Val Thr Leu Lys Met Ala
    130                 135                 140

Ala Asp Ala Arg Tyr Tyr Arg Ile Lys Ser Ser Gln Ala Thr Asn Pro
145                 150                 155                 160

Thr Tyr Gln Met Ser Glu Leu Gly Asp Ala Phe Thr Tyr Gly Glu Ser
                165                 170                 175

Ala Ala Tyr Val Val Leu Phe Gly Asp Lys Glu Ser Gln Thr Val Pro
            180                 185                 190

Arg Ser Trp Val Glu Trp Leu Phe Glu Lys Glu Gln Leu Pro Gln His
        195                 200                 205

Leu Gly Trp Lys Arg Pro Ala Thr Ser Phe Glu Leu Asn Asp Leu Asp
    210                 215                 220

Lys Phe Met Ala Leu Ile Gln Asn Tyr Thr Gln Glu Ile Glu Glu Pro
225                 230                 235                 240

Ser Cys Glu Ser Arg Lys Gln Arg Arg Lys Pro Arg Gly Pro Ser His
                245                 250                 255

Phe Gly Phe

<210> 2
<211> 261
<212> PRT
<213> Humicola insolens

<400> 2

Gly Phe His Asp Trp Glu Pro Pro Gly Pro Asn Asp Val Arg Ala Pro
1               5                   10                  15

Cys Pro Met Leu Asn Thr Leu Ala Asn His Gly Phe Leu Pro His His
            20                  25                  30

Gly Arg Asp Leu Thr Arg Lys Gln Val Val Asp Gly Leu Tyr Asn Gly
        35                  40                  45

Leu Asn Ile Asn Lys Thr Ala Ala Ser Thr Leu Phe Asp Phe Ala Leu
    50                  55                  60

Met Thr Ser Pro Lys Pro Asn Ala Thr Thr Phe Ser Leu Asp Asp Leu
65                  70                  75                  80

Gly Arg His Asn Ile Leu Glu His Asp Ala Ser Leu Ser Arg Thr Asp
                85                  90                  95

Ala Tyr Phe Gly Asp Val Leu Ala Phe Asn Lys Thr Ile Phe Glu Glu
            100                 105                 110

Thr Lys Arg His Trp Gly Lys Ser Pro Ile Leu Asp Val Thr Ala Ala
        115                 120                 125

Ala Arg Ala Arg Leu Gly Arg Ile Gln Thr Ser Lys Ala Thr Asn Pro
    130                 135                 140

Glu Tyr Phe Met Ser Glu Leu Gly Asn Ile Phe Thr Tyr Gly Glu Ser
145                 150                 155                 160

Val Ala Tyr Ile Met Leu Ile Gly Asp Ala Lys Thr Gly Arg Ala Asn
                165                 170                 175

Arg Arg Trp Val Glu Tyr Trp Phe Glu Asn Glu Arg Leu Pro Thr His
            180                 185                 190

Leu Gly Trp Arg Arg Pro Ser Lys Glu Leu Thr Ser Asp Val Leu Asp
        195                 200                 205

Thr Tyr Ile Ser Leu Ile Gln Asn Ile Thr Leu Thr Leu Pro Gly Gly
    210                 215                 220

Thr Asp Pro Val Lys Arg Arg Ala Ala Ser His Phe Val Phe Pro Phe
225                 230                 235                 240

Gly Gln Gly Leu Gly Gly Pro Ala Gly Val Ala Leu Met Leu Ile Ser
                245                 250                 255

Val Val Ala Tyr Gly
            260

<210>  3
<211>  371
<212>  PRT
<213>  Agrocybe aegerita

<400>  3

Met Lys Tyr Phe Pro Leu Phe Pro Thr Leu Val Phe Ala Ala Arg Val
1               5                   10                  15

Val Ala Phe Pro Ala Tyr Ala Ser Leu Ala Gly Leu Ser Gln Gln Glu
            20                  25                  30

Leu Asp Ala Ile Ile Pro Thr Leu Glu Ala Arg Glu Pro Gly Leu Pro
        35                  40                  45

Pro Gly Pro Leu Glu Asn Ser Ser Ala Lys Leu Val Asn Asp Glu Ala
    50                  55                  60

His Pro Trp Lys Pro Leu Arg Pro Gly Asp Ile Arg Gly Pro Cys Pro
65                  70                  75                  80

Gly Leu Asn Thr Leu Ala Ser His Gly Tyr Leu Pro Arg Asn Gly Val
                85                  90                  95

Ala Thr Pro Val Gln Ile Ile Asn Ala Val Gln Glu Gly Leu Asn Phe
            100                 105                 110

Asp Asn Gln Ala Ala Val Phe Ala Thr Tyr Ala Ala His Leu Val Asp
        115                 120                 125

Gly Asn Leu Ile Thr Asp Leu Leu Ser Ile Gly Arg Lys Thr Arg Leu
    130                 135                 140

Thr Gly Pro Asp Pro Pro Pro Pro Ala Ser Val Gly Gly Leu Asn Glu
145                 150                 155                 160

His Gly Thr Phe Glu Gly Asp Ala Ser Met Thr Arg Gly Asp Ala Phe
                165                 170                 175

Phe Gly Asn Asn His Asp Phe Asn Glu Thr Leu Phe Glu Gln Leu Val
            180                 185                 190

Asp Tyr Ser Asn Arg Phe Gly Gly Gly Lys Tyr Asn Leu Thr Val Ala
        195                 200                 205

Gly Glu Leu Arg Phe Lys Arg Ile Gln Asp Ser Ile Ala Thr Asn Pro
    210                 215                 220

Asn Phe Ser Phe Val Asp Phe Arg Phe Phe Thr Ala Tyr Gly Glu Thr
225                 230                 235                 240

Thr Phe Pro Ala Asn Leu Phe Val Asp Gly Arg Arg Asp Asp Gly Gln
                245                 250                 255

Leu Asp Met Asp Ala Ala Arg Ser Phe Phe Gln Phe Ser Arg Met Pro
            260                 265                 270

Asp Asp Phe Phe Arg Ala Pro Ser Pro Arg Ser Gly Thr Gly Val Glu
        275                 280                 285

Val Val Ile Gln Ala His Pro Met Gln Pro Gly Arg Asn Val Gly Lys
    290                 295                 300

Ile Asn Ser Tyr Thr Val Asp Pro Thr Ser Ser Asp Phe Ser Thr Pro
305                 310                 315                 320

Cys Leu Met Tyr Glu Lys Phe Val Asn Ile Thr Val Lys Ser Leu Tyr
                325                 330                 335

Pro Asn Pro Thr Val Gln Leu Arg Lys Ala Leu Asn Thr Asn Leu Asp
            340                 345                 350

Phe Phe Phe Gln Gly Val Ala Ala Gly Cys Thr Gln Val Phe Pro Tyr
        355                 360                 365

Gly Arg Asp
    370

## Description

### FIELD AND OBJECT OF THE INVENTION

The process of the present invention relates to the biocatalytic transformation of isophorone (3,5,5-trimethylcyclohex-2-en-1-one; also known as α-isophorone) and 4-hydroxyisophorone (4HIP) by several fungal unspecific peroxygenases (UPOs). The recently discovered peroxygenases from the ascomycetes *Chaetomium globosum* (*Cgl*UPO) and *Humicola insolens* (recombinant protein, r*Hin*UPO) were able to selectively oxidize isophorone to form the high value products for the pharmaceutical and flavour and fragrance industry 4HIP and 4-ketoisophorone (4KIP), and to oxidize 4HIP to form 4KIP. The well-known peroxygenase from the basidiomycete *Agrocybe aegerita* (*Aae*UPO) was found to be less regioselective, forming 7-hydroxyisophorone (7HIP) and 7-formylisophorone (7FIP) in addition to 4HIP. However, *Aae*UPO is the most stereoselective peroxygenase yielding the S-enantiomer of 4HIP (with 88% enantiomeric excess, *ee*). Moreover, using r*Hin*UPO full kinetic resolution of racemic HIP can be obtained in only 15 min reactions, with >75% recovery of the *R*-enantiomer. These biocatalysts, that only require H₂O₂ or organic peroxides for activation, potentially represent a novel simple and environmentally friendly route for the production of isophorone derivatives.

### BACKGROUND ART

3,5,5-Trimethyl-4-hydroxy-2-cyclohexen-1-one (4-hydroxyisophorone, 4HIP) is a saffron constituent described in literature as flavour and aromatizing compound in food and tobacco products (patent US4898984, patent US6469215) and is an intermediate in the synthesis of pharmaceuticals (patent CH549961) and natural pigments (O. Isler, H. Lindlar, M. Montavon, R. Rüegg, G. Saucy, P. Zeller, HCA 1956, 39, 2041-2053.). Moreover, the flavour and fragrance structurally similar 3,5,5-trimethyl-2-cyclohexen-1,4-dione (4-ketoisophorone, 4KIP) is also an important intermediate in the synthesis of vitamins, like vitamin E, and carotenoids (M. Eggersdorfer, D. Laudert, U. Létinois, T. McClymont, J. Medlock, T. Netscher, W. Bonrath, Angew. Chem. Int. Ed. 2012, 51, 12960-12990).

A wide variety of chemical methods are available for the synthesis of 4HIP and 4KIP. Both were usually synthesized from β-isophorone (3,5,5-trimethylcyclohex-3-en-1-one) (patent US3944620) with the previous isomerization of isophorone to β-isophorone, but the rearrangement of β-isophorone to isophorone is a main drawback of these processes. The direct oxidation of isophorone to 4KIP with molecular oxygen appeared as a solution, using as catalysts 'copper (II) chloride-acetylacetone' or molybdenum-based catalytic systems (W. Zhong, L. Mao, Q. Xu, Z. Fu, G. Zou, Y. Li, D. Yin, H. Luo, S. R. Kirk, Appl. Cat. A:General 2014, 486, 193-200) with the requirements of toxic heavy-metal catalyst and the production of non-desired side products. However, a direct chemical oxidation process of isophorone to 4HIP is not available in literature and this compound is usually synthesized by reduction of 4KIP [(M. Ishihara, T. Tsuneya, H. Shiota, M. Shiga, K. Nakatsu, J. Org. Chem. 1986, 51, 491-495); (M. Hennig, K. Püntener, M. Scalone, Tetrahedron: Asymmetry 2000, 11, 1849-1858), which can be expensive as starting material.

Enzymes are promising biocatalysts for hydroxylation reactions, and several biotransformation processes for the synthesis of 4HIP and 4KIP have been described. For example, the isophorone microbial biotransformation was described by fungus like *Aspergillus niger* (Y. Mikami, Y. Fukunaga, M. Arita, Y. Obi, T. Kisaki, Agric. Biol. Chem. 1981, 45, 791-793) or *Alternaria alternata* and *Neurospora crassa* (I. Kiran, O. Ozsen, T. Celik, S. Ilhan, B. Y. Gürsu, F. Demirci, Natural Product Communications 2013, 8, 59-61). In both studies, 4HIP and 7HIP were found as main metabolites. More recently, a biotransformation process using a recombinant *Escherichia coli* strain transformed with the P450-BM3 gene, together with a NADPH dehydrogenase gene, allowed the more selective production of 4HIP on a kilogram scale (I. Kaluzna, T. Schmitges, H. Straatman, D. van Tegelen, M. Müller, M. Schürmann, D. Mink, Org. Process Res. Dev. 2016, 20, 814-819) and 4KIP was produced in one-pot two step or with a whole-cell cascade using both recombinant P450-WAL and Cm-ADH10 (M. Tavanti, F. Parmeggiani, J. R. G. Castellanos, A. Mattevi, N. J. Turner, ChemCatChem 2017, 9, 3338-3348). However, cytochromes P450 present the disadvantages of their higher instability due to their intracelular nature, and the frequent need of auxiliary enzymes/domains and expensive cofactors.

Fungal peroxygenases, described as unspecific peroxygenases (UPOs; EC.1.11.2.1) in IUBMB Enzyme Nomenclature, are novel and appealing biocatalysts for organic synthesis since its simplicity (only requires H₂O₂ for activation) circumvent mayor disadvantages of cytochromes P450 while catalyzing the same kind of oxyfunctionalization reactions (Y. Wang, D. Lan, R. Durrani, F. Hollmann, Curr. Opin. Chem. Biol. 2017, 37, 1-9). The first enzyme of this class was discovered in 2004 in the basidiomycete *Agrocybe aegerita* and since then new fungal peroxygenases came out from *Coprinellus radians, Marasmius rotula* and more recently, from the ascomycete *Chaetomium globosum.* Their widespread occurrence in the fungal kingdom was demonstrated during the analysis of basidiomycete and ascomycete genomes, in which several-thousand peroxygenase-type genes were identified (M. Hofrichter, H. Kellner, M. J. Pecyna, R. Ullrich. Adv. Exp. Med. Biol. 2015, 851, 341-368) allowing for the production of recombinant enzymes like the one of *Coprinosis cinerea* or *Humicola insolens,* heterologously expressed by Novozymes A/S in *Aspergillus oryzae.* The spectrum of reactions catalyzed by these enzymes is increasing and includes oxygenation reactions on aromatic (C. Aranda, R. Ullrich, J. Kiebist, K. Scheibner, J. C. del Río, M. Hofrichter, A. T. Martínez, A. Gutiérrez, Catal. Sci. Technol. 2018, 8, 2394-2401; M. Hofrichter and R. Ullrich, Curr. Opin. Chem. Biol. 2014, 19, 116-125) and aliphatic compounds (A. Olmedo, C. Aranda, J. C. del Río, J. Kiebist, K. Scheibner, A. T. Martínez and A. Gutiérrez, Angew. Chem. Int. Ed. 2016, 55, 12248-12251; E. D. Babot, J. C. del Río, L. Kalum, A. T. Martínez and A. Gutiérrez, Biotechnol. Bioeng. 2013, 110, 2332; A. Gutiérrez, E. D. Babot, R. Ullrich, M. Hofrichter, A. T. Martínez and J. C. del Río, Arch. Biochem. Biophys. 2011, 514, 33-43; S. Peter, M. Kinne, X. Wang, R. Ulrich, G. Kayser, J. T. Groves and M. Hofrichter, FEBS J. 2011, 278, 3667-3675), including fatty acids epoxidation (C. Aranda, A. Olmedo, J. Kiebist, K. Scheibner, J. C. del Río, A. T. Martínez, A. Gutiérrez, ChemCatChem 2018, 10, 3964-3968) and chain shortening (A. Olmedo, J. C. del Río, J. Kiebist, K. Scheibner, A. T. Martínez and A. Gutiérrez, Chem. -Eur. J. 2017, 23, 16985-16989), and also reactions of rather complex substrates like steroids (E. D. Babot, J. C. del Río, M. Cañellas, F. Sancho, F. Lucas, V. Guallar, L. Kalum, H. Lund, G. Gröbe, K. Scheibner, R. Ullrich, M. Hofrichter, A. T. Martínez and A. Gutiérrez, Appl. Environ. Microbiol. 2015, 81, 4130-4142; R. Ullrich, M. Poraj-Kobielska, S. Scholze, C. Halbout, M. Sandvoss, M. J. Pecyna, K. Scheibner and M. Hofrichter, J. Inorg. Biochem. 2018, 183, 84-93) and secosteroids (F. Lucas, E. D. Babot, J. C. del Río, L. Kalum, R. Ullrich, M. Hofrichter, V. Guallar, A. T. Martínez and A. Gutiérrez, Catal. Sci. Technol. 2016, 6, 288-295; E. D. Babot, J. C. del Río, L. Kalum, A. T. Martínez and A. Gutiérrez, ChemCatChem 2015, 7, 283-290) in which hydroxylation or side chain removal occurred.

In view of the background art, it is concluded that there is a need for a direct oxidation of isophorone or 4HIP to 4KIP, and that fungal peroxygenases can be of high interest for such reactions.

### BRIEF DESCRIPTION OF THE INVENTION

It is the object of the present invention a process for selective synthesis of 4-hydroxyisophorone (4HIP) and 4-ketoisophorone (4KIP), characterized in that the process is catalyzed by a fungal peroxygenase, classified as unspecific peroxygenase EC 1.11.2.1 in IUBMB Enzyme Nomenclature.

In preferred embodiments the fungal peroxygenase is selected from the ascomycetes *Chaetomium globosum* Kunze or *Humicola insolens* Cooney & R. Emers (also known as *Thermomyces lanugimosus* Tsiki) or from the basidiomycete *Agrocybe aegerita* (V. Brig.) Singer (also known as *Cyclocybe aegerita* [V. Brig] Vizzini), or from related fungal species. The fungal peroxygenase can be a wild enzyme obtained from cultures of the above fungi or a recombinant enzyme from expression hosts, including but not exclusively *Aspergillus* species, *Pichia* and other yeast species, *Trichoderma* species and *Escherichia* coli and other bacterial species. Alternatively, the fungal peroxygenase from the three previously mentioned fungi may be a mutated variant from *Chaetomium globosum, Humicola insolens* or *Agrocybe aegerita* obtained by chemical mutagenesis or protein engineering methods.

In a more preferred embodiment, the fungal peroxygenase has more than 60% identity, or preferably more than 80% identity, or more preferably more than 95% identity, even more preferably is identical with the *Chaetomium globosum* sequence included as SEQ ID NO1. In an optional more preferred embodiment, the fungal peroxygenase has more than 60% identity, or preferably more than 80% identity, or more preferably more than 95% identity, even more preferably is identical with the *Humicola insolens* sequence included as SEQ ID NO 2. In another optional more preferred embodiment, the fungal peroxygenase has more than 60% identity, or preferably more than 80% identity, or more preferably more than 95% identity, even more preferably is identical with the *Agrocybe aegerita* sequence included as SEQ ID NO 3.

Isophorone and 4HIP can be used as enzyme substrate. When the process is carried out with isophorone as substrate and with *Aae*UPO as fungal peroxygenase, stereoselectivity is improved enabling to obtain the S-enantiomer of 4HIP with at least 88% enantiomeric excess. When the process is carried out with racemic 4HIP as substrate and with r*Hin*UPO or *Cgl*UPO as fungal peroxygenase, selective oxidation of the S-enantiomer to 4KIP is produced enabling isolation of the *R*-enantiomer of 4HIP, with at least 60% recovery.

Preferentially, the process comprises the following steps: **i)** Solving the substrate in a solvent selected from acetone, acetonitrile, water and combinations thereof with a final concentration of the substrate in the solvent ranging from 0.01 mM to 1 M, preferably from 0.1mM to 100 mM; **ii)** Adding the substrate solution from the previous step to a reaction mixture comprising the fungal peroxygenase, at a final concentration in the reaction mixture ranging from 5 nM to 100 µM, in water or buffer of pH 4-10, and an oxidizing agent selected from H₂O₂ and organic peroxides, at a final concentration in the reaction mixture ranging from 0.25 mM to 1 M; and **iii)** Allowing the mixture from the previous step to react at a temperature ranging between 15ºC and 60 ºC, during a period ranging from 1 min to 96 h. Alternatively, the process is carried out in pure isophorone without solvent (neat conditions) comprising the following steps: i) adding pure isophorone to a reaction mixture comprising the fungal peroxygenase, at a final concentration in the reaction mixture ranging from 5 nM to 100 µM, in water or buffer of pH 4-10, and an oxidizing agent selected from H₂O₂ and organic peroxides, at a final concentration in the reaction mixture ranging from 0.25 mM to 1 M; ii) allowing the mixture from the previous step to react at a temperature ranging between 15ºC and 60 ºC, during a period ranging from 1 min to 96 h.

### BRIEF DESCRIPTION OF THE FIGURES

**Fig. 1****.** GC-MS analysis of 0.1 mM isophorone reactions (30 min) with 0.1 µM of *Mro*UPO, r*Cci*UPO, *Cgl*UPO, r*Hin*UPO, *Aae*UPO and control without enzyme, showing the remaining substrate (IP, isophorone) and the hydroxylated (4HIP and 7HIP) and oxo (4KIP and 7FIP) derivatives.
**Fig. 2****.** Time course of 10 mM isophorone (IP) reaction with 5 µM *Cgl*UPO (**A**) and r*Hin*UPO (**B**) and H₂O₂ (added with a syringe pump to give 5 mM/h concentration), showing substrate and products (4HIP and 4KIP) concentrations.
**Fig. 3****.** Chiral HPLC analysis of 15 min reactions of 4HIP (racemic mixture including the [*R*] and [*S*] enantiomers) as substrate with r*Hin*UPO (**B**), *Cgl*UPO (**C**) and *Aae*UPO (**D**), resulting in deracemization (enabling *R*-HIP recovery) due to S-HIP oxidation to 4KIP (**B**), and control without enzyme (**A**).

### DETAILED DESCRIPTION OF THE INVENTION AND EMBODIMENTS

### Enzymes

*Aae*UPO (isoform II, 46 kDa), the first UPO described in 2004 (R. Ullrich and M. Hofrichter, FEBS Lett. 2005, 579, 6247-6250), is a wild-type (i.e. non-recombinant) peroxygenase purified from cultures of *A. aegerita* TM-A1, grown in soybean-peptone medium. *Mro*UPO is another wild-type peroxygenase (32 kDa) purified from cultures of *M. rotula* DSM-25031 (German Collection of Microorganisms and Cell Cultures, Braunschweig). *Cgl*UPO (36 kDa) is a third wild-type peroxygenase recently purified from cultures of *C. globosum* DSM-62110.

The r*Cci*UPO (44 kDa) and r*Hin*UPO recombinant enzymes were provided by Novozymes A/S. r*Cci*UPO corresponds to the protein model 7249 from the sequenced *C. cinerea* genome available at the JGI (http://genome.jgi.doe.gov/Copci1) used in several studies (E. D. Babot, J. C. del Río, L. Kalum, A. T. Martínez and A. Gutiérrez, Biotechnol. Bioeng. 2013, 110, 2332; E. D. Babot, J. C. del Río, M. Cañellas, F. Sancho, F. Lucas, V. Guallar, L. Kalum, H. Lund, G. Gröbe, K. Scheibner, R. Ullrich, M. Hofrichter, A. T. Martínez and A. Gutiérrez, Appl. Environ. Microbiol. 2015, 81, 4130-4142; E. D. Babot, J. C. del Río, L. Kalum, A. T. Martínez and A. Gutiérrez, ChemCatChem 2015, 7, 283-290). The r*Hin*UPO sequence has been more recently reported (Patent US 9908860 B2) and used for oxyfunctionalizations that are not catalyzed by other UPOs. Both UPOs were expressed in *Aspergillus oryzae* (patent WO/2008/119780).

The amino-acid sequences of *Cgl*UPO, r*Hin*UPO and *Aae*UPO are included as SEQ ID NO 1, SEQ ID NO 2 and SEQ ID NO 3, respectively.

All UPOs were purified from fast protein liquid chromatography using a combination of size exclusion and ion exchange columns. Purification was confirmed by sodium dodecylsulfate-polyacrylamide gel electrophoresis and UV-visible spectra with characteristic maximum at 418 nm (Soret band of heme-thiolate proteins). Enzyme concentration was estimated from the characteristic spectrum of peroxidase complex with carbon monoxide.

### Model compounds

3,5,5-Trimethyl-2-cyclohexen-1-one (isophorone; also known as α-isophorone) from Sigma Aldrich (97% purity) was tested as substrate of the above UPOs. 3,5,5-trimethyl-2-cyclohexen-1,4-dione (4KIP) also from Sigma Aldrich and chemically-synthesized 4-hydroxy-3,5,5-trimethyl-2-cyclohexen-1-one (4HIP), were used as standards in gas chromatography-mass spectrometry (GC-MS) analyses. 4HIP, obtained as a racemic mixture by chemical reduction of 4KIP, was used as substrate in enzymatic reactions together with isophorone.

### Enzyme reactions

Reactions (1-mL volume) with isophorone (0.1 mM) were performed at 30 ºC, pH 7, in 50 mM phosphate buffer. The enzyme concentrations ranged from 50 nM to 10 µM, using 2.5 to 5 mM H₂O₂. In control experiments, substrates were treated under the same conditions (including H₂O₂) but without enzyme. After 30 min reaction, products were extracted with methyl *tert*-butyl ether, which was evaporated under N2 and the products dissolved in chloroform to be analyzed by GC-MS.

Reactions at higher isophorone concentration (10 mM) were performed with *Cgl*UPO and r*Hin*UPO (2-5 µM). The H₂O₂ was added with a syringe pump to give a concentration in the reaction of 1 or 5 mM per hour.

Reactions for chiral analyses were carried out with 10 mM isophorone or 4HIP (racemic mixture) and 5 µM enzyme (*Cgl*UPO, r*Hin*UPO and *Aae*UPO) for 60 min, with H₂O₂ manually added in doses to a final concentration of 5-20 mM. Products were extracted with ethyl acetate and directly analyzed by GC-MS or dried and dissolved in the mobile phase to be analyzed by HPLC.

### Enzyme kinetics

Reactions were carried out with 6.25-6400 µM substrate and 100 nM enzyme. They were initiated adding 0.5 mM H₂O₂ and stopped by vigorous shaking in 5 mM sodium azide. Reaction times in which the reaction velocity was linear were previously selected: 5 min for *Aae*UPO, 3 min for *Cgl*UPO and 1 min for r*Hin*UPO. All reactions were performed in triplicates. Product quantification was carried out by GC-MS using external standard curves, and kinetic parameters - turnover number (*k*_{cat}), Michaelis constant (*K*ₘ) and catalytic efficiency (*k*_{cat}/*K*ₘ) - were obtained by fitting the data to the Michaelis-Menten equation, or the corresponding variation of this equation when substrate inhibition is occurring, using SigmaPlot (Systat Softwarwe Inc., San Jose, CA, USA).

### GC-MS analysis

The analyses were performed with a Shimadzu GC-MS QP 2010 Ultra system, using a fused-silica DB-5HT capillary column (30 m × 0.25 mm internal diameter, 0.1 µm film thickness) from J&W Scientific. The oven was heated from 50 °C (1.5 min) to 90 °C (2 min) at 30 °C min⁻¹, and then from 90 °C to 250 °C (15 min) at 8 °C min⁻¹. The injection was performed at 250 °C and the transfer line was kept at 300 °C. Compounds were identified by comparing their mass spectra and retention times with those of the available authentic standards and with the NIST library.

### Chiral HPLC

Chiral analyses were performed in a Shimadzu LC-2030C 3D system equipped with a photo-diode array detector using a chiral column Chiralpak IG (5 µM particle size, 4.6 mm diameter x 150 mm, Daicel Chemical Industries Ltd.) equipped with a Chiralpak IG guard column (5 µM particle size, 4.0 mm diameter x 10 mm). The column was eluted in isocratic mode with 95% hexane and 5% isopropanol at 0.5 mL/min for 60 min and the absorbance was monitored at 254 nm. Enantiomers were identified based on the elution order previously reported (S. Dezvarei, J. H. Z. Lee, S. G. Bell, Enzyme Microb. Technol. 2018, 111, 29-37)

### Regioselectivity in isophorone transformation by different UPOs

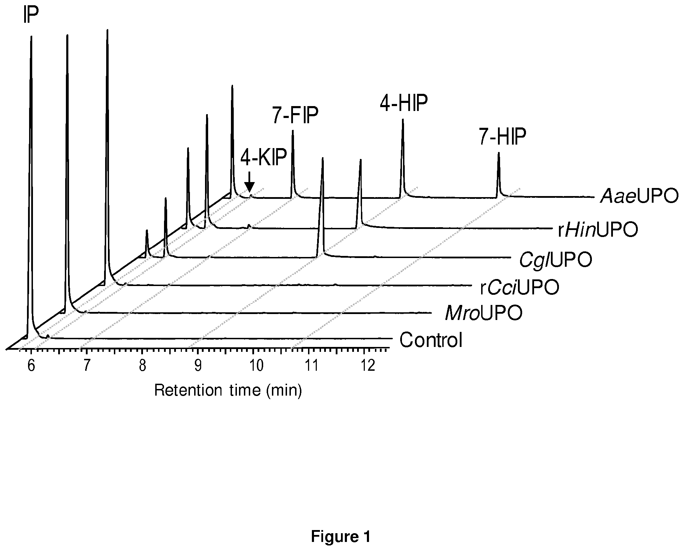

In the present patent, the ability of several UPOs to oxidize isophorone was analyzed, and different transformation patterns were observed (Figure 1). The peroxygenases from the ascomycetes *C. globosum* (*Cgl*UPO) and *H. insolens* (r*Hin*UPO), and the basidiomycete *A. aegerita* (*Aae*UPO) were found to transform the substrate, although with different regioselectivities (Table 1). However, the enzymes from the basidiomycetes *C. cinerea* (r*Cci*UPO) and *M. rotula* (*Mro*UPO) were unable to modify the substrate, even at the highest enzyme doses tested.

**Table 1. Comparison of several fungal peroxygenases in isophorone (0.1 mM) conversion and relative abundance (% of total) of products at 30 min enzymatic reaction.**

| | Conversion | 4HIP | 7HIP | 4KIP | 7FIP |
|---|---|---|---|---|---|
| *Aae*UPO^{a,e} | 69 | 46 | 25 | 1 | 28 |
| *Aae*UPO^{b,e} | 91 | 45 | 19 | 1 | 35 |
| *Cgl*UPO^{a,e} | 89 | 76 | - | 23 | 1 |
| *Cgl*UPO^{c,f} | 100 | - | - | 100 | - |
| r*Hin*UPO^{a,e} | 75 | 52 | - | 47 | 2 |
| r*Hin*UPO^{c,f} | 100 | 3 | | 97 | - |
| r*Cci*UPO^{d,e} | 0 | - | - | - | - |
| *Mro*UPO^{d,e} | 0 | - | - | - | - |

| | | | | | |
|---|---|---|---|---|---|
| ^{a}0.1 µM, ^{b}0.25 µM, ^{c}0.5 µM, ^{d}10 µM, ^{e}2.5 mM H₂O₂, ^{f}5 mM H₂O₂ | | | | | |

*Cgl*UPO and r*Hin*UPO selectively oxidized isophorone in position 4 yielding the monohydroxylated 4HIP and the keto 4KIP derivatives (Scheme below):

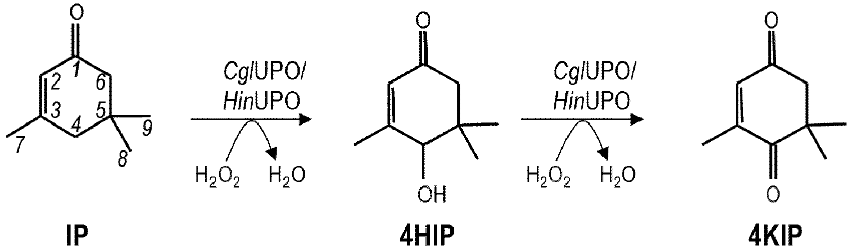

The above compounds were identified by their retention times compared with authentic standard and mass spectra. Besides the 4HIP and 4KIP molecular ions at *m*/*z* 154 and 152, respectively, their mass spectra showed a shift from isophorone fragments at *m*/*z* 54 and 82 to *m*/*z* 70 and 98 in 4HIP and to *m*/*z* 68 and 96 in 4KIP, which corresponds to the insertion of a hydroxyl or oxo group. While isophorone conversion by r*Hin*UPO and *Cgl*UPO (84 and 95%, respectively) was high at the lower enzyme dose (0.1 µM), the former UPO showed more tendency to overoxidize 4HIP into 4KIP, as shown by the lower ratio between both products (1:1) compared to the latter enzyme (4:1) (Table 1). *Aae*UPO was found to be less selective oxidizing isophorone since in addition to 4HIP (and traces of 4KIP), other monohydroxylated and keto derivatives were formed (Scheme below):

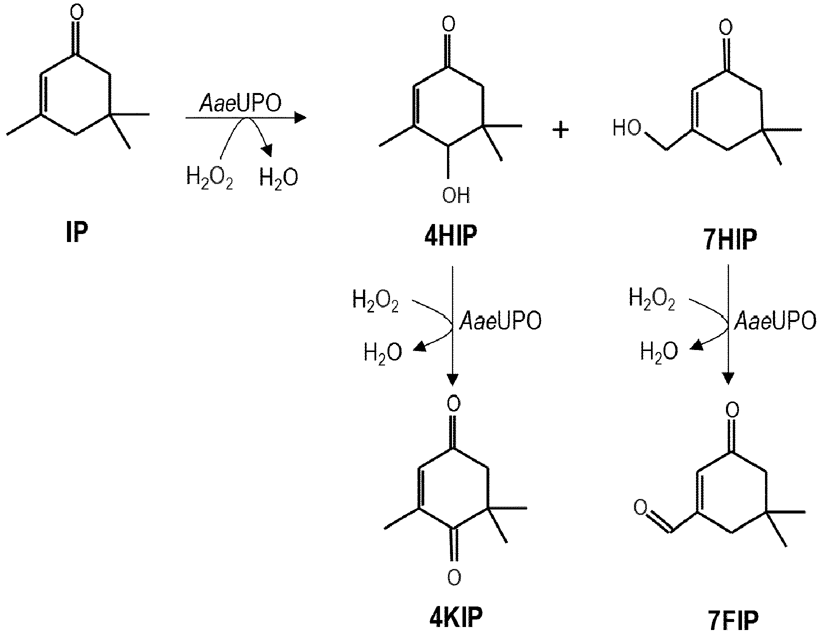

These side products were identified as 7HIP and 7FIP since their mass spectra matched with those previously reported, with the singular mass fragment at *m*/*z* 125 in 7HIP, different from that at *m*/*z* 112 in 4HIP. Reactions with low *Aae*UPO dose (0.1 µM) showed that hydroxylation takes place in similar proportion in C₄ (46%) and C₇ (54%), but 7HIP was rapidly overoxidized to form the aldehyde, and 7FIP and 4HIP are the main final products of the reaction (Table 1) since 4HIP was not oxidized to 4KIP.

The regioselectivity observed in the hydroxylation of isophorone by some UPOs is similar to that observed with P450s. Among them, P450cam-RhFRed variants have been reported to yield 4HIP, 7HIP and isophorone oxide (2,3-epoxy-3,5,5-trimethyl-1-cyclohexanone) as major products, with 4HIP as the only product from one of the variants. 4HIP was also the main product of reactions with CYP102A1 and CYP101A1, although minor amounts of the epoxide, 7HIP and further oxidation products were observed. However, P450s, unlike r*Hin*UPO or *Cgl*UPO, seem unable to oxidize 4HIP into 4KIP and, therefore, two enzymes (a P450 and an alcohol dehydrogenase) are necessary to obtain 4KIP from isophorone, while only one enzyme is used in the present patent application.

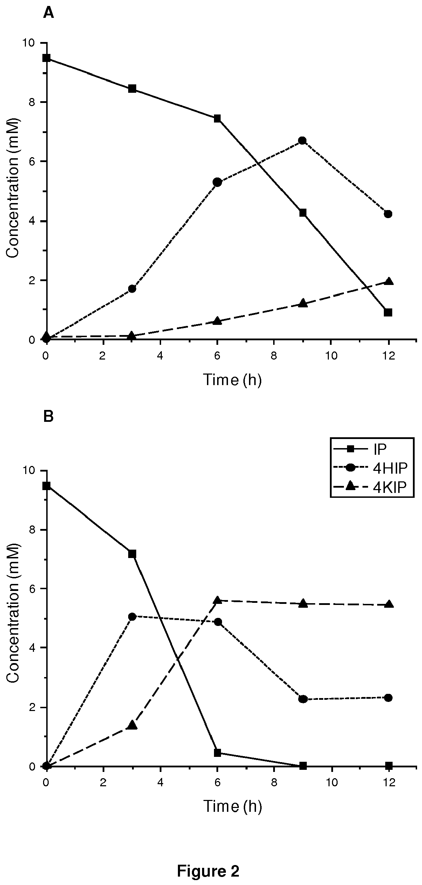

In view of the higher selectivity to form the products of interest 4HIP and 4KIP, *Cgl*UPO and r*Hin*UPO were selected to perform reactions with higher (x100) substrate loads (Figure 2). These experiments revealed a faster substrate conversion by r*Hin*UPO that completely transformed isophorone in 6 h, while *Cgl*UPO needed 12 h for 87% conversion. Additionally, a higher proportion of 4KIP was observed in the r*Hin*UPO reactions. A higher enzyme dose will be needed for complete conversion into 4KIP, as found when lower substrate concentrations are used (Table 1).

### Kinetics of isophorone hydroxylation by UPOs

Despite the difficulties for determination of enzyme initial reaction rates by GC-MS, kinetic curves for isophorone hydroxylation by the three UPOs could be obtained and reaction parameters (*k*_{cat}, *K*ₘ and *k*_{cat}/*K*ₘ) were estimated (Table 2).

**Table 2. Estimated kinetic parameters for isophorone hydroxylation by several fungal peroxygenases (AaeUPO, CglUPO and rHinUPO). Data represent mean values of three replicates with standard errors**

| | *k*_{cat} (s⁻¹) | *K*ₘ (µM) | *k*_{cat}/ *K*ₘ (M⁻¹·s⁻¹) |
|---|---|---|---|
| *Aae*UPO | 20.1±1.1 | 1381.3±196.7 | 1.4±0.1 x 10⁴ |
| *Cgl*UPO | 3.8±0.1 | 243.3±19.8 | 1.6±0.1 x 10⁴ |
| r*Hin*UPO | 23.3±1.4 | 287.5±39.9 | 8.1±1.2 x 10⁴ |

There were differences in enzyme affinities since the *K*ₘ values were four- and two-fold higher for *Aae*UPO than for *Cgl*UPO and r*Hin*UPO, revealing the higher isophorone affinity of the two latter enzymes. Moreover, r*Hin*UPO presented a ten-fold higher turnover number (*k*_{cat}) compared to *Cgl*UPO, which resulted in five-fold higher catalytic efficiency, while the *Aae*UPO and *Cgl*UPO efficiencies were similar.

The catalytic efficiency of these UPOs hydroxylating isophorone is in the range of the previously reported for hydroxylation of cyclohexane by other UPOs. On the other hand, the *K*ₘ of *Cgl*UPO and r*Hin*UPO for isophorone hydroxylation was similar of the ones reported (380-440 µM) for CYP102A1 variants when decoy molecules were used, and the turnover numbers of these variants (2.5-5.5 s⁻¹) were also similar to the one herein reported for *Cgl*UPO (4.4 s⁻¹).

Higher total turnover numbers (TTNs) were attained with r*Hin*UPO (2660) than with *Cgl*UPO (1820) under the same reaction conditions, although these values could be up to 5500 and 3600 when lower enzyme dose was used (Table 3).

**Table 3. Product formation and catalytic performance of CglUPO and rHinUPO in isophorone hydroxylation after 9 h enzymatic reaction**

| | 4HIP (mM) | 4KIP (mM) | TTN | TOF (min⁻¹) |
|---|---|---|---|---|
| *Cgl*UPO | 6.7 | 1.2 | 1820 | 3.4 |
| r*Hin*UPO | 2.3 | 5.5 | 2660 | 4.9 |

The TTNs are in the range of those reported for two-step (1567) or one-step (3421) isophorone oxidation by the combination of P450-WAL and Cm-ADH10.

### Peroxygenase enantioselectivity on isophorone and racemic 4HIP

Enantioselectivity in the synthesis of 4HIP by the three UPOs hydroxylating isophorone was determined by HPLC. The results of the chiral analysis showed that only the reaction with *Aae*UPO can be considered as stereoselective, with an ee of 88% *S*-4HIP (Table 4), while the ee in *Cgl*UPO and r*Hin*UPO was of 40% and 4%, respectively. The three UPOs formed the *S*-enantiomer preferentially, in contrast with P450s that form the *R*-enantiomer.

**Table 4. Results from chiral HPLC analysis of 10 mM isophorone reaction (60 min) with AaeUPO, CglUPO and rHinUPO (5 µM) showing the yield of the R and S enantiomers of 4HIP, and the resulting enantiomeric excess (ee), together with the amount of 4KIP formed and the total conversion yield (% of substrate) under the present conditions.^{a,b}**

| | Conversion (%) | 4HIP (mM) | 4KIP (mM) | *R*-4HIP (%) | *S*-4HIP (%) | *ee* (%) |
|---|---|---|---|---|---|---|
| *Aae*UPO ^{a} | 51 | 2.5 | 0.1 | 6 | 94 | 88 |
| *Cgl*UPO 3^{a} | 24 | 3.3 | 0.3 | 30 | 70 | 40 |
| r*Hin*UPO ^{b} | 36 | 2.0 | 0.4 | 48 | 52 | 4 |

| | | | | | | |
|---|---|---|---|---|---|---|
| ^{a,b} Reactions using 20 and 5 mM H₂O₂, respectively | | | | | | |

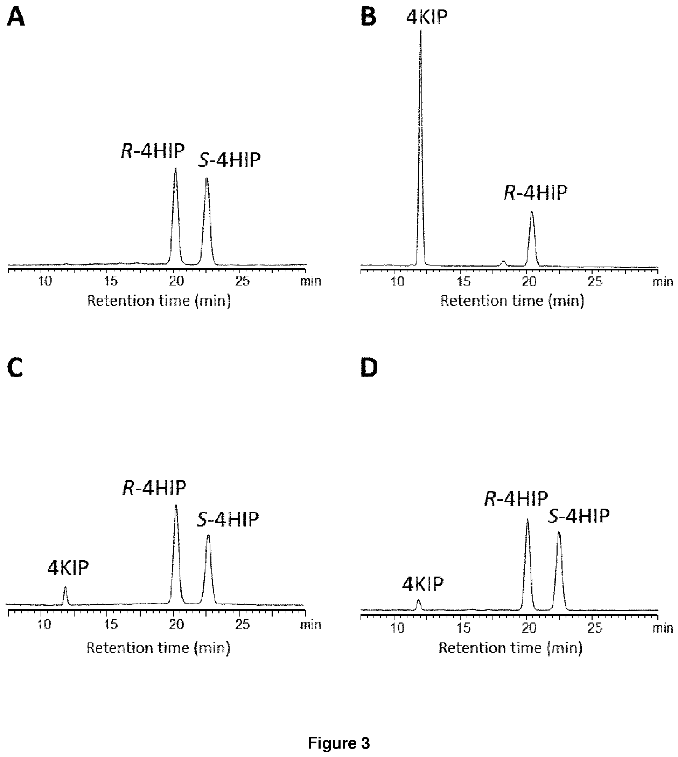

The above values were estimated in reaction conditions where the product concentration was similar for all the enzymes (2.0-3.3 mM) and 4HIP overoxidation was minimal, since it was observed that the *ee* changed as 4KIP was formed. This was due to higher velocity converting the *S*-enantiomer compared to the *R*-enantiomer as observed in r*Hin*UPO reactions with racemic 4HIP as substrate (Figure 3). In this way, a kinetic resolution of the racemate, with *ee* of 100% and 99% and 60-70% recovery, can be achieved with r*Hin*UPO and *Cgl*UPO respectively. In contrast, *Aae*UPO slowly converted the racemic mixture of 4HIP, as also observed in the reaction with isophorone where only traces of 4KIP were formed (Figure 1), and no enantiomeric enrichment is produced.

### ACKNOWLEDGEMENT

The project leading to this application has received funding from the European Union's Horizon 2020 research and innovation programme under grant agreement H2020-BBI-PPP-2015-2-1-720297.

## Claims

1. Process for selective synthesis of 4-hydroxyisophorone and 4-ketoisophorone, **characterized in that** the process is catalyzed by a fungal peroxygenase, classified as unspecific peroxygenase EC 1.11.2.1 in IUBMB Enzyme Nomenclature.

2. Process according to claim 1, wherein the fungal peroxygenase is selected from the ascomycetes *Chaetomium globosum* or *Humicola insolens* or from the basidiomycete *Agrocybe aegerita.*

3. Process according to claim 2, wherein the fungal peroxygenase is a wild enzyme obtained from cultures of *Chaetomium globosum, Humicola insolens* or *Agrocybe aegerita.*

4. Process according to claim 2, wherein the fungal peroxygenase is a recombinant enzyme from *Chaetomium globosum, Humicola insolens* or *Agrocybe aegerita* obtained by heterologous expression in an expression host.

5. Process according to claims 3 and 4, wherein the fungal peroxygenase is a mutated variant of the enzyme from *Chaetomium globosum, Humicola insolens* or *Agrocybe aegerita* obtained by chemical mutagenesis or protein engineering.

6. Process according to anyone of claims 1 and 5, wherein the fungal peroxygenase has more than 60% identity, or preferably more than 80% identity, or more preferably more than 95% identity, even more preferably is identical with the *Chaetomium globosum* sequence included as SEQ ID NO1.

7. Process according to anyone of claims 1 to 5, wherein the fungal peroxygenase has more than 60% identity, or preferably more than 80% identity, or more preferably more than 95% identity, even more preferably is identical with the *Humicola insolens* sequence included as SEQ ID NO 2.

8. Process according to anyone of claims 1 to 5, wherein the fungal peroxygenase has more than 60% identity, or preferably more than 80% identity, or more preferably more than 95% identity, even more preferably is identical with the *Agrocybe aegerita* sequence included as SEQ ID NO 3.

9. Process according to anyone of claims 1 to 8, wherein isophorone is used as enzyme substrate.

10. Process according to anyone of claims 1 to 8 wherein 4-hydroxyisophorone is used as enzyme substrate.

11. Process according to claim 9, wherein the reaction of isophorone with *Aae*UPO is stereoselective, enabling to obtain the S-enantiomer of 4-hydroxyisophorone with at least 88% enantiomeric excess.

12. Process according to claim 10, wherein the reaction of racemic 4-hydroxyisophorone with r*Hin*UPO or *Cgl*UPO enables full kinetic resolution of the racemic mixture yielding the *R*-enantiomer of 4-hydroxyisophorone.

13. Process for selective synthesis of 4-hydroxyisophorone or 4-ketoisophorone by a fungal peroxygenase according to anyone of claims 1 to 12, comprising the following steps:
- solving the substrate in a solvent selected from acetone, acetonitrile, water and combinations thereof with a final concentration of the substrate in the solvent ranging from 0.01 mM to 1 M, preferably from 0.1 mM to 100 mM.
- adding the substrate solution from the previous step to a reaction mixture comprising the fungal peroxygenase, at a final concentration in the reaction mixture ranging from 5 nM to 100 µM, in water or buffer of pH 4-10, and an oxidizing agent selected from H₂O₂ and organic peroxides, at a final concentration in the reaction mixture ranging from 0.25 mM to 1 M.
- allowing the mixture from the previous step to react at a temperature ranging between 15ºC and 60 ºC, during a period ranging from 1 min to 96 h.

14. Process for selective synthesis of 4-hydroxyisophorone or 4-ketoisophorone by a fungal peroxygenase according to anyone of claims 1 to 12, comprising the following steps:
- adding pure isophorone to a reaction mixture comprising the fungal peroxygenase, at a final concentration in the reaction mixture ranging from 5 nM to 100 µM, in water or buffer of pH 4-10, and an oxidizing agent selected from H₂O₂ and organic peroxides, at a final concentration in the reaction mixture ranging from 0.25 mM to 1 M.
- allowing the mixture from the previous step to react at a temperature ranging between 15ºC and 60 ºC, during a period ranging from 1 min to 96 h.
